# FASCICULE DE BREVET EUROPEEN

(11) **EP 0 702 025 B1**
(45) Date de publication et mention de la délivrance du brevet: **19.12.2001**
(21) Numéro de dépôt: 95402013.7
(22) Date de dépôt: 06.09.1995
(51) Int. Cl.: C07J 3/00, C07J 41/00

(54) **Procédé de préparation d'un dérivé stéroide 17bêta-carboxy et nouveaux intermédiaires**
Verfahren zur Herstellung 17beta-carboxy Steroidderivate und neue Zwischenprodukte
Process for the preparation of 17beta-carboxy steroids and new intermediates

(30) Priorité: 06.09.1994 FR 9410661
(43) Date de publication de la demande: 20.03.1996
(73) Titulaire: Aventis Pharma S.A., 92160 Antony (FR)
(72) Inventeur: Roussel, Patrick, F-94320 Thiais (FR); Vivat, Michel, F-77400 Lagny Sur Marne (FR)

(56) Documents cités:
- EP-A- 0 042 606
- EP-A- 0 289 327
- JOURNAL OF CHEMICAL SOCIETY, CHEMICAL COMMUNICATIONS, no. 1, pages 25-26, A. KUREK ET AL 'Synthesis of 3beta-methoxy-5alpha,14alpha-card-20(22)-e nolide from 3beta-methoxy-5alpha-androstan-17-one. A method for the construction of the cardenolide side-chain.'

## Description

La présente invention concerne un procédé de préparation d'un dérivé stéroïde 17β-carboxy et les nouveaux intermédiaires obtenus.

L'invention a pour objet un procédé de préparation de l'acide de formule (I) : 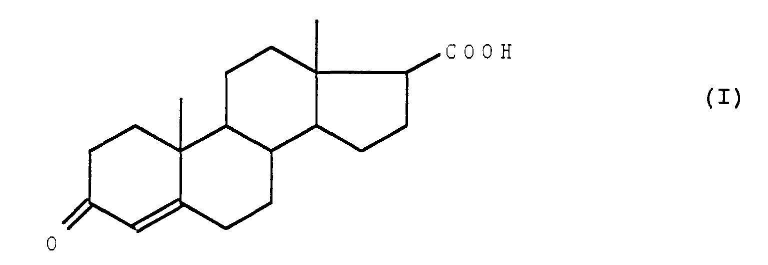 caractérisé en ce que l'on soumet un composé de formule (II) 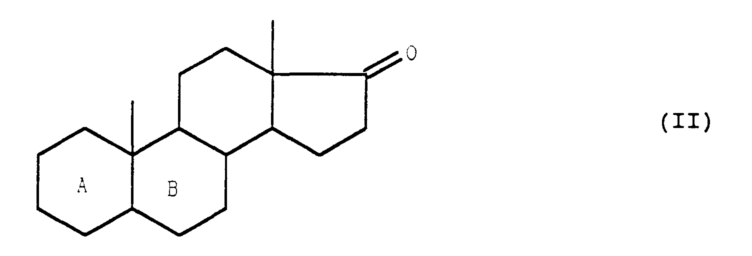 dans laquelle les cycles A et B représente un reste : 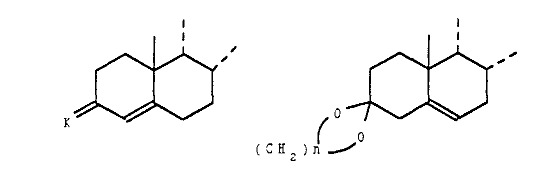 ou 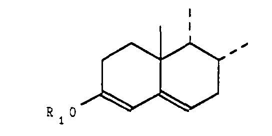 dans lequel K représente un groupement protecteur du radical oxo de formule : 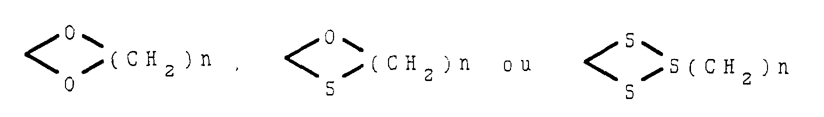 où n est égal à 2 ou 3, et R₁ représente un reste éther ou ester, à l'action d'un halonitrile, en présence d'une base, pour obtenir un composé de formule (III) : 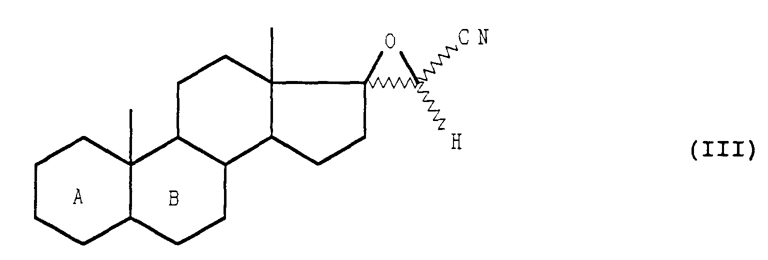 dans laquelle les cycles A et B ont la signification indiquée précédemment et les traits ondulés symbolisent un mélange d'isomères, dont on libère la fonction cétone en position 3 pour obtenir un composé de formule (IV) : 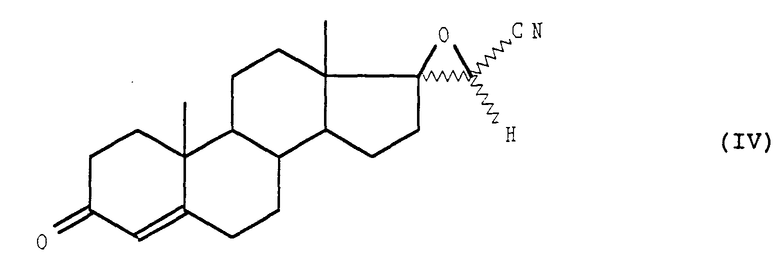 dans laquelle les traits ondulés ont la signification indiquée précédemment,
que l'on traite par un hydracide de formule HX dans lequel X représente un atome d'halogène, en milieu anhydre, pour obtenir un composé de formule (V) : 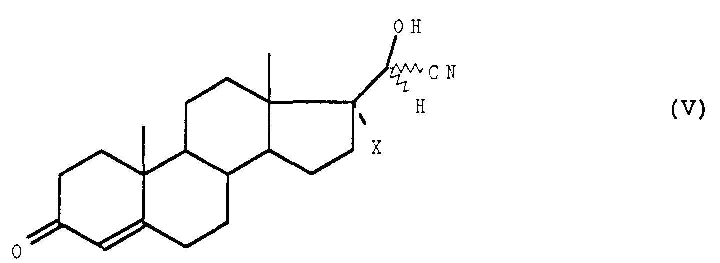 dans laquelle X et les traits ondulés ont la signification indiquée précédemment,
dont on protège la fonction hydroxy sous forme d'ester pour obtenir un composé de formule (VI) : 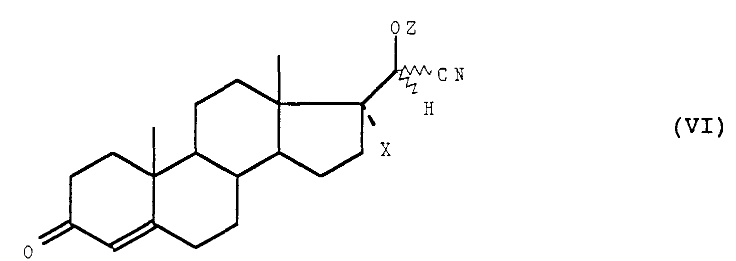 dans laquelle Z représente le reste d'un groupement ester protecteur du radical hydroxy et X et les traits ondulés ont la signification précédemment,
que l'on soumet à l'action d'un agent de déshydrohalogénation pour obtenir le composé de formule (VII) : 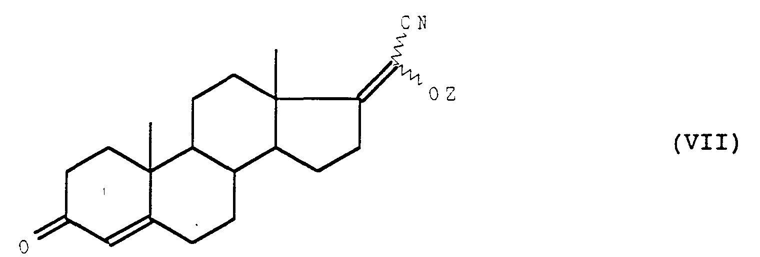 dans laquelle Z et les traits ondulés ont la signification indiquée précédémment, que l'on soumet à une hydrolyse alcaline puis à un traitement acide pour obtenir le composé de formule (I) attendu.

Lorsque R₁ représente un éther, il peut s'agir de tout reste connu de l'homme du métier pour bloquer la position 3 sous cette forme et notamment il peut s'agir d'un radical alkyle renfermant de 1 à 6 atomes de carbone, d'un radical alkoxyalkoxyalkyle renfermant de 3 à 8 atomes de carbone, d'un radical aryle renfermant de 6 à 10 atomes de carbone ou d'un radical aralkyle renfermant de 7 à 12 atomes de carbone.

Lorsque R₁ représente un radical alkyle, il s'agit par exemple d'un radical méthyle, éthyle, propyle, isopropyle, n-butyle, sec-butyle, tert-butyle, pentyle ou hexyle.

Lorsque R₁ représente un radical alkoxyalkoxyalkyle, il s'agit par exemple d'un radical méthoxyéthoxyméthyle.

Lorsque R₁ représente un radical aralkyle, il s'agit par exemple d'un radical benzyle ou phénéthyle.

Lorsque R₁ représente un radical aryle, il s'agit par exemple d'un radical phényle ou d'un radical phényl substitué, en particulier par un ou plusieurs radicaux alkyles.

Lorsque R₁ représente un reste éther, il peut encore s'agir d'un groupement silylé, par exemple d'un groupement trialkylsilyle tel que triméthylsilyle, tert-butyl, diméthyl-silyle ou encore par exemple d'un groupement triarylsilyle tel que triphénylsilyle ou diarylalkylsilyle tel que diphényl tert-butylsilyle.

Lorsque R₁ représente un reste ester, il peut s'agir de tout reste connu de l'homme du métier pour bloquer la position 3 sous cette forme et notamment il peut s'agir d'un reste -COR₁, R₁ étant un radical alkyle, aryle ou aralkyle tel que défini ci-dessus.

L'invention a notamment pour objet un procédé tel que défini précédemment caractérisé en ce que l'on utilise au départ un composé de formule (II) dans laquelle les cycles A et B représentent un reste 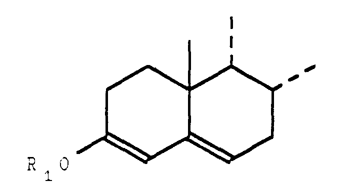 dans laquelle R₁ est défini comme précédemment et particulièrement un radical alkyle renfermant de 1 à 6 atomes de carbone.

L'halonitrile que l'on fait agir sur le composé de formule (II) peut être le bromoacétonitrile ou particulièrement le chloroacétonitrile. On opère en présence d'une base forte, de préférence non nucléophile, telle qu'un alcoolate, un hydrure, un amidure ou un hydroxyde de métal alcalin, ou encore un organolithien, par exemple le méthylate, l'éthylate, le terbutylate ou le teramylate de sodium ou potassium, le disisopropylamidure de lithium, ou encore la soude ou la potasse.

Il peut être avantageux d'opérer en phases hétérogènes en présence d'un catalyseur de transfert de phase, lequel peut être notamment un sel d'ammonium quaternaire, par exemple le bromure de tétrabutylammonium, le chlorure de triéthylbenzylammonium ou le chorure de tricaprylméthylammonium, ou un sel de phosphonium.

On utilise de préférence un alcoolate tel que le terbutylate ou le teramylate de potassium ou de sodium.

La réaction est effectuée sur sein d'un solvant organique tel que le toluène, le dichlorométhane, le diméthoxyéthane, le diméthylformamide, le tétrahydrofuranne ou d'un mélange de ces solvants, par exemple le mélange toluène/tétrahydrofuranne ou encore d'un mélange terbutanol/tétrahydrofuranne.

La libération de la fonction cétone en position 3 est effectuée par des moyens appropriés à la nature du groupement protecteur. On utilise un agent acide en présence d'eau ou d'un mélange eau-alcanol, dans le cas d'un cétal. Il s'agit par exemple d'un acide minéral ou organique tel que l'acide chlorhydrique, bromhydrique, sulfurique, perchlorique, nitrique, paratoluène sulfonique, acétique, formique, oxalique ou d'un mélange d'acides, ou encore d'un résine acide, par exemple une résine sulfonique. Dans le cas d'un thiocétal ou d'un cétal mixte, la déprotection de la fonction 3-oxo est réalisée par action de l'iode en présence d'une base par exemple un bicarbonate alcalin, ou par action de l'iode en quantité catalytique, en présence d'un agent oxydant, notamment l'eau oxygénée, par action de l'iodure de méthyle, de l'acide glyoxylique, ou encore de sels de métaux, tels le mercure ou le cadmium. On peut, en général, opérer dans un solvant tel qu'un alcanol inférieur, par exemple le méthanol ou l'éthanol, en mélange avec un solvant halogéné, par exemple le chlorure de méthylène, en présence d'eau. Dans le cas d'un cétal mixte, la déprotection est encore effectuée par exemple par un sel mercurique tel que le chlorure mercurique en présence d'un tampon acide acétique/acétate de potassium à 100°C environ, par le nickel de Raney dans les mêmes conditions que ci-dessus ou par le mélange acide chlorhydrique-acide acétique à chaud.

Dans le cas où R₁ représente un reste éther ou ester, on utilise également un traitement acide dans les conditions décrites ci-dessus pour le cétal. On peut utiliser par exemple l'acide acétique ou sulfurique ou un mélange de ces acides.

L'hydracide que l'on utilise pour l'ouverture de la fonction époxyde peut être par exemple l'acide bromhydrique ou de préférence, l'acide chlorhydrique. La réaction est effectuée de préférence en milieu anhydre au sein d'un solvant organique de préférence peu ou non polaire comme par exemple le diméthoxyéthane, l'éther éthylique, l'acétate d'éthyle, le dichlorométhane ou encore le toluène, ces deux derniers étant préférés.

On opère avantageusement en présence d'une amine tertiaire telle que la pyridine ou la triéthylamine.

La protection de la fonction hydroxy en position 20 sous forme d'ester peut être réalisée selon les méthodes usuelles en utilisant un dérivé d'acide carboxylique et notamment un halogénure ou un anhydride d'acide alcanoïque ou aromatique, en opérant de préférence en présence d'une base azotée. On peut utiliser par exemple l'ahydride acétique, propionique, valérique ou benzoïque, en présence de pyridine.

La déshydrohologénation est effectuée par action d'une base telle que par exemple une amine tertiaire comme la triéthylamine ou la pyridine, ou encore d'une base non nucléophile telle que le carbonate de lithium-bromure de lithium, la soude ou la potasse. La réaction est effectuée au sein d'un solvant organique non hydrophile tel que par exemple le dichlorométhane, le diméthylformamide, le diméthylsulfoxyde ou, de préférence, le toluène. On opére avantageusement au reflux du milieu réactionnel.

L'hydrolyse alcaline est effectuée au moyen d'une base forte notamment l'hydroxyde de baryum, l'hydroxyde de potassium, la carbonate de sodium, ou de préférence l'hydroxyde de sodium. On opére en présence d'eau ou d'un mélange eau-alcool qui stabilise le sel de base formé, le cas échéant en système double phase, avec le solvant dans lequel l'étape précédénte a été effectuée.

L'isolement de l'acide oxoétiocholénique est réalisé par traitement acide du sel de base selon les méthodes usuelles, par exemple au moyen d'un acide minéral tel que l'acide sulfurique ou l'acide chlorhydrique.

L'invention a notamment pour objet un procédé tel que défini précédemment, caractérisé en ce que l'on opère sans isoler intermédiairement les composés de formule (IV) et suivantes.

L'invention a aussi pour objet à titre de produits industriels nouveaux et notamment à titre d'intermédiaires nécessaires à la mise en oeuvre du procédé de l'invention :
- les composés de formule (F1) : 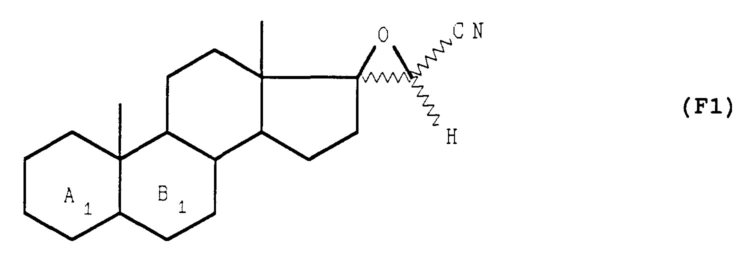
dans laquelle les cycles A1 et B1 ont la signification indiquée précédemment pour les cycles A et B, ou représentent le reste 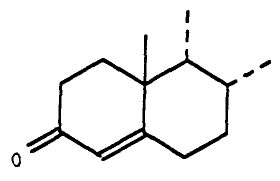 et les traits ondulés ont la signification indiquée précédemment,
- les composés de formule (F2) : 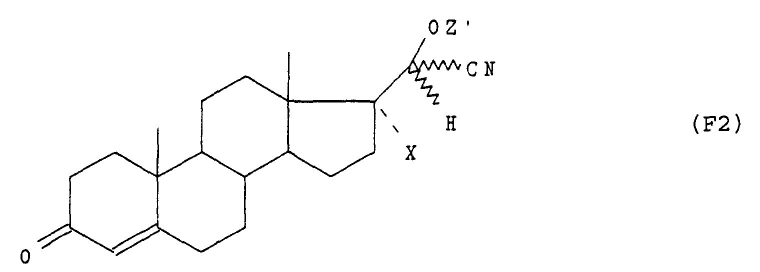 dans laquelle X et les traits ondulés ont la signification indiquée précédemmment et Z' représente un atome d'hydrogène ou un groupement ester protecteur du radical hydroxy,
- les composés de formule (VII) : 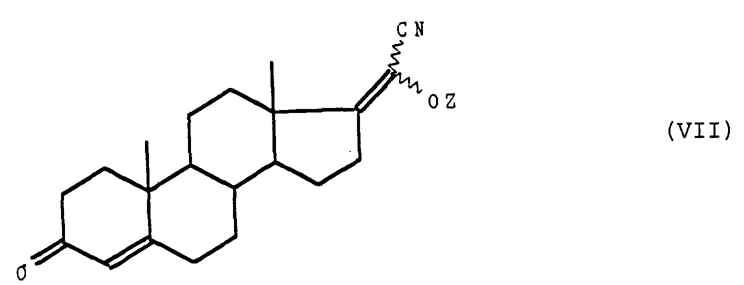 dans laquelle z et les traits ondulés ont la signification indiquée précédemment.

La référence J. Chem. Soc. décrit la synthèse, au départ de stéroides 17-oxo, de stéroides comportant en 17 une chaine de type cardenolide, de formule 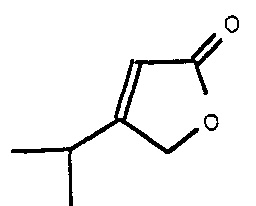 par une suite de réactions mettant en jeu lesdits composés 17 oxo dans une condensation de type Knoevenagel et générant notamment des intermédiaires de formule 20-cyano 21-OH/OAc que l'on pourrait rapprocher des composés de formule VI. Cependant, lesdites réactions sont différentes de celles de notre demande et il s'en suit qu'aucun intermédiaire n'est commun aux deux synthèses. De plus, les composés finals sont également différents.

Le composé de formule (II) est décrit dans le brevet français 1.563.607.

L'acide de formule (I) ou acide oxoétiocholénique est un produit connu notamment comme intermédiaire de synthèse et décrit, par exemple, dans la demande européenne 562.849.

Les exemples suivants illustrent l'invention sans toutefois la limiter.

### EXEMPLE 1 : 17β-carboxy 3-oxo androst-4-ène : acide oxoetiocholénique

### STADE A : 17,20-époxy 3-méthoxy 17α-pregna 3,5-diène-21-nitrile

On refroidit à -45°C, sous gaz inerte, 15 g de 3-méthoxy androsta-3,5-dièn-17-one dans 120 cm³ de tétrahydrofuranne et 5,5 g de terbutanol puis ajoute 9 g de terbutylate de potassium puis, en 4 heures à environ -45°C, 4,8 g de chloroacétonitrile en solution dans 30 cm³ de tétrahydrofuranne.

On introduit le mélange réactionnel sous gaz inerte, dans une solution refroidie à 0°C comprenant 2 g de chlorure d'ammonium dans 300 cm³ d'eau puis maintient 30 minutes sous agitation. On filtre, lave au méthanol à 20 % d'eau, reprend le résidu dans 15 cm³ d'un mélange isopropanol/éther isopropylique 1-2, essore, lave de nouveau avec 30 cm³ du mélange ci-dessus. On essore, sèche sous pression réduite et obtient 13,4 g de produit attendu. F = 180°C.

| Spectre IR (CHCl₃) | |
|---|---|
| C=C conj. | 1654-1629 cm⁻¹ |
| C≡N | 2248 cm⁻¹ |

| Spectre RMN (CDCl₃ ppm) | |
|---|---|
| 0,94 | CH₃ en 18 |
| 0,98 | CH₃ en 19 |
| 3,49 | CH en 20 |
| 3,58 | CH₃ en 3 |
| 5,14 (d) | CH en 4 |
| 5,25 (d) | CH en 6 |

### STADE B : 17β-carboxy 3-oxo androst-4-ène : acide oxoetiocholénique

### 1) Hydrolyse de l'éther d'énol en position 3

A 12,85 g de 17,20-époxy 3-méthoxy 17α-pregna 3,5-diène 21-nitrile préparé comme indiqué au stade A. On ajoute sous atmosphère inerte, 64 cm³ d'acide acétique, 13 cm³ d'eau et 13 cm³ d'acide sulfurique dilué (2N).

On agite 1 heure et demie à température ambiante, ajoute en 10 minutes, 64 cm³ de toluène et 64 cm³ d'eau, décante, lave la phase toluénique à l'eau, puis avec une solution aqueuse de bicarbonate de sodium à 2 %, et distille le toluène sous 150/100 mbars jusqu'à un volume d'environ 25 cm³.

### 2) Ouverture de l'époxyde

On ajoute sous atmosphère inerte 100 cm³ de dichlorométhane et 6 cm³ de pyridine au milieu réactionnel ci-dessus, refroidit à -5°C, introduit de l'acide chlorhydrique gazeux pendant 3 heures environ, maintient sous agitation pendant 1 heure à -5°C et neutralise l'excès d'acide chlorhydrique par addition de 21,5 cm³ de pyridine.

### 3) Acétylation de la chlorhydrine

On laisse revenir la température de la suspension préparée ci-dessus à + 10°C environ, puis ajoute en 30 minutes 7,8 cm³ d'anhydride acétique et 6 cm³ de pyridine. On agite 16 heures à température ambiante, lave à l'eau, décante, distille le résidu de 400 à 150 mbars jusqu'à l'obtention d'un produit brut cristallisé.

### 4) Elimination 17-20 du dérivé acétate

On ajoute sous atmosphère inerte 50 cm³ de toluène et 25 cm³ de triéthylamine au produit obtenu précédemment, chauffe au reflux pendant 3 heures et demie et laisse revenir à température ambiante.

### 5) Hydrolyse de l'acétate d'énol

On ajoute sous atmosphère inerte 50 cm³ d'eau et 25 cm³ de soude au milieu réactionnel préparé précédemment et agite 20 heures, ajoute 250 cm³ d'eau et décante la phase aqueuse.

### 6) Acidification du sel de sodium/préparation de l'acide

On refroidit à +10°/+15°C sous atmosphère d'azote la phase aqueuse obtenue précédemment et ajoute 80 cm³ d'acide sulfurique dilué (4N) sous agitation. On chasse l'acide cyanhydrique gazeux par balayage de gaz inerte, essore le précipité formé, le lave à l'eau, le sèche sous pression réduite et obtient 9,33 g de produit attendu.
F = 255-256°C.

| Spectre IR (CHCl₃) | |
|---|---|
| C=O | 1702 cm⁻¹ |
| Δ₄-3-one | 1662-1615 cm⁻¹ |

| Spectre RMN (CDCl₃ ppm) | |
|---|---|
| 0,79 | CH₃ en 18 |
| 1,19 | CH₃ en 19 |
| 5,74 | CH en 4 |

### EXEMPLE 2 : 17β-carboxy 3-oxo androst-4-ène : acide oxoetiocholénique

### STADE A : 17,20-époxy 3-oxo 17α-pregn-4-ène-21-nitrile

On mélange 3 heures à 20°C sous gaz inerte 1,017 g du produit obtenu au stade A de l'exemple 1, dans 10 cm³ d'acide acétique et 5 cm³ d'eau, ajoute 4 cm³ de tétrahydrofuranne et agite 2 heures.

On évapore les solvants sous pression réduite, chromatographie le résidu sur silice (éluant toluène-acétate d'éthyle 8-2) et obtient 691 mg de produit attendu.

| Spectre IR (CHCl₃) | |
|---|---|
| C≡N | 2240 cm⁻¹ |
| C=O | 1662 cm⁻¹ |
| C=C | 1617 cm⁻¹ |

| Spectre RMN (CDCl₃ ppm) | |
|---|---|
| 0,95 | CH₃ en 18 |
| 1,20 | CH₃ en 19 |
| 3,32-3,48 | CH en 20 |
| 5,75 | CH en 4. |

### STADE B1 : 17α-chloro 20-hydroxy 3-oxo pregn-4-ène-21-nitrile

A 2 cm³ d'une solution 4,7 N d'acide chlorhydrique gazeux dans le diméthoxy éthane 4,7 N, refroidie à +5°/+10°C, on ajoute 1 g du produit obtenu comme indiqué au stade A, agite 3 heures, ajoute de nouveau 1 cm³ de réactif, agite 1 heure en maintenant la température à 10°C et utilise le milieu réactionnel tel quel pour le stade suivant.

### STADE B2 : 17α-chloro 20-acétoxy 3-oxo pregn-4-ène-21-nitrile

On agite sous atmosphère inerte 0,25 g d'époxyde préparé au stade A, en solution dans 10 cm³ de dichlorométhane et 0,1 cm³ de pyridine.

On introduit lentement à 20°C environ pendant 1 heure, un courant d'acide chlorhydrique gazeux et élimine l'excès d'acide chlorhydrique par un balayage de gaz inerte.

La moitié du milieu réactionnel est utilisé telle quelle pour le stade suivant.

L'autre moitié du milieu réactionnel est lavée à l'eau ; on le sèche, évapore le solvant sous pression réduite, reprend le résidu dans 2 cm³ d'un mélange éthanol-eau (1-1), triture, essore, lave à l'eau, sèche sous pression réduite et obtient 0,1 g de produit attendu. F = 230°-240°C (décomp.).

| Spectre RMN (CDCl₃ ppm) | |
|---|---|
| 0,97-0,98 | CH₃ en 18 |
| 1,21 | CH₃ en 19 |
| 3,05-3,22 | OH en 20 |
| 4,56-4,77 | CH en 20 |
| 5,75 | CH en 4. |

### STADE C : 20-acétoxy 17α-chloro 3-oxo pregn-4-ène-21-nitrile

On ajoute au milieu réactionnel obtenu au stade B1 (ou au cours du stade B), refroidi à 10°C, 3 cm³ de diméthoxy-éthane puis ajoute en quelques minutes 1,7 cm³ de pyridine et 1 cm³ d'anhydride acétique. On laisse revenir à température ambiante en 1 heure, ajoute 0,8 cm³ de pyridine et 5 cm³ d'eau, abandonne à +4°C pendant 16 heures, essore le précipité formé, le lave au diméthoxyéthane, le sèche sous pression réduite et recueille 0,364 g de produit attendu. F = 238°C.

| Spectre IR (CHCl₃) | |
|---|---|
| OAc | 1757 cm⁻¹ |
| Δ₄-3-one | 1665-1616 cm⁻¹ |

| Spectre RMN (CDCl₃ ppm) | |
|---|---|
| 1,04 | CH₃ en 18 |
| 1,21 | CH₃ en 19 |
| 2,22 | O-Ac |
| 5,74 | CH en 4 et en 20. |

### STADE D : (E+Z) 20-acétoxy 3-oxo pregn-4,17(20)-diène-21-nitrile

A 100 mg de produit obtenu au stade C, sous atmosphère inerte, on ajoute 0,5 cm³ de toluène et 0,2 cm³ de triéthylamine. On chauffe au reflux pendant 5 heures, refroidit vers 10°C, essore le précipité, le lave au toluène puis à l'eau, le sèche sous pression réduite et recueille 91 mg de produit attendu. F = 185-190°C.

| Spectre IR (CHCl₃) | |
|---|---|
| C≡N (conj) | 2223 cm⁻¹ |
| OAc | 1773 cm⁻¹ |
| Δ₄-3-one | 1662-1616 cm⁻¹ |

| Spectre RMN (CDCl₃ ppm) | |
|---|---|
| 0,97 | CH₃ en 18 |
| 1,19 | CH₃ en 19 |
| 2,20 | O-Ac |
| 5,74 | CH₃ en 4. |

### STADE E : 17β-carboxy 3-oxo androst-4-ène : acide oxoetiocholénique

En opérant comme aux stades B5 et B6 de l'exemple 1, en utilisant au départ le produit obtenu au stade D ci-dessus, on obtient l'acide oxoetiocholénique attendu.

## Revendications

1. Procédé de préparation de l'acide oxoétiocholénique de formule (I) : 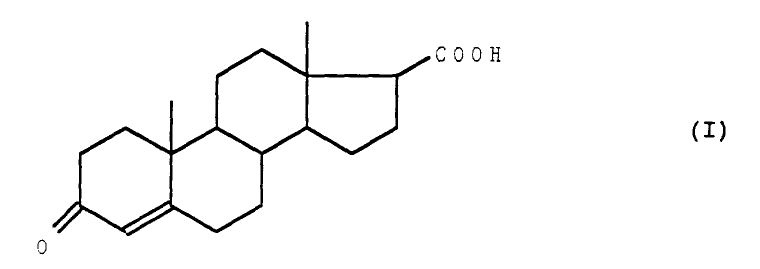 **caractérisé en ce que** l'on soumet un composé de formule (II) 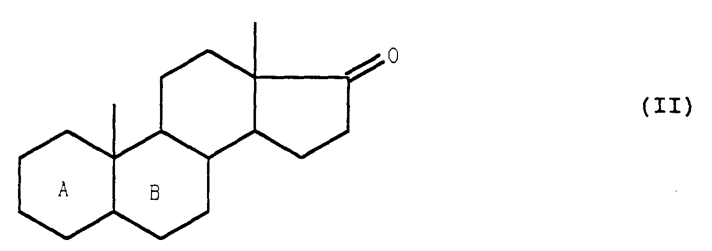 dans laquelle les cycles A et B représente un reste : 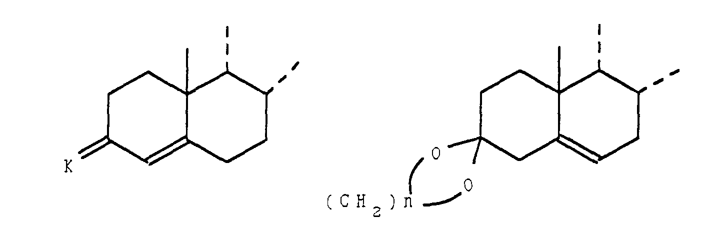 ou 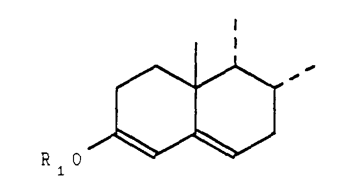 dans lequel K représente un groupement protecteur du radical
oxo de formule : 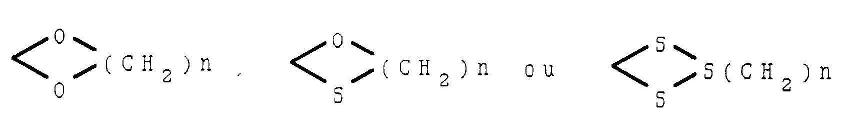 où n est égal à 2 ou 3, et R₁ représente un reste éther ou ester, à l'action d'un halonitrile, en présence d'une base, pour obtenir un composé de formule (III) : 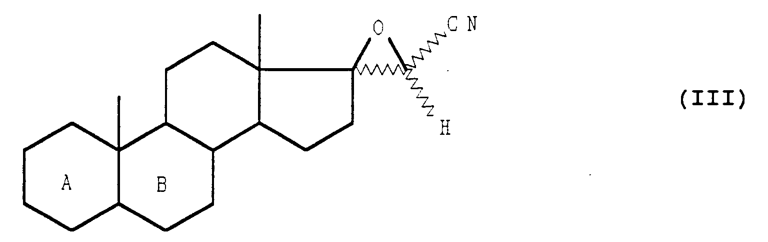 dans laquelle les cycles A et B ont la signification indiquée précédemment et les traits ondulés symbolisent un mélange d'isomères, dont on libère la fonction cétone en position 3 pour obtenir un composé de formule (IV) : 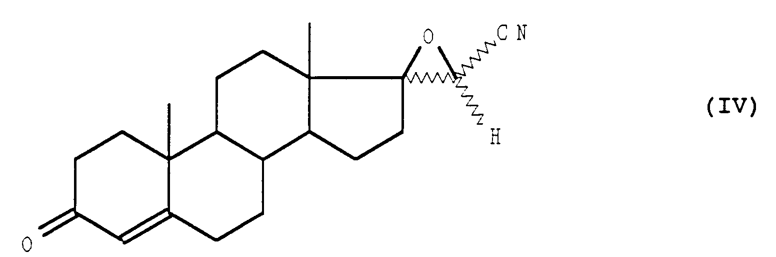 dans laquelle les traits ondulés ont la signification indiquée précédemment,
que l'on traite par un hydracide de formule HX dans lequel X représente un atome d'halogène, en milieu anhydre, pour obtenir un composé de formule (V) : 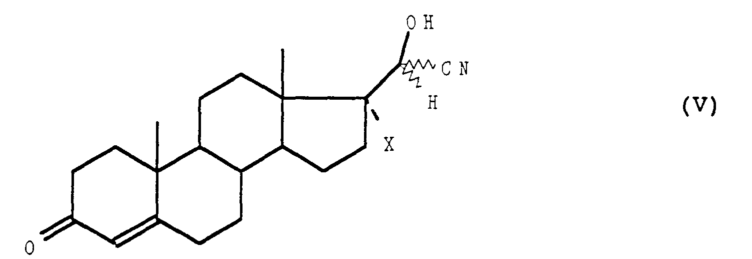 dans laquelle X et les traits ondulés ont la signification indiquée précédemment,
dont on protège la fonction hydroxy sous forme d'ester pour obtenir un composé de formule (VI) : 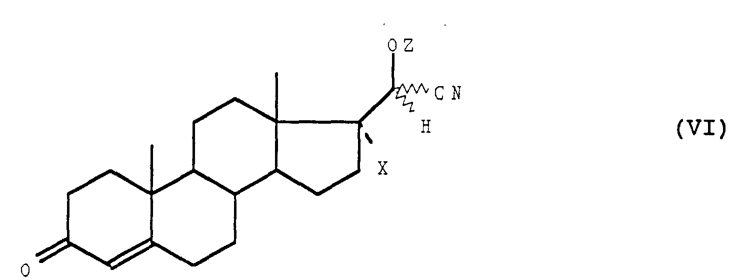 dans laquelle Z représente le reste d'un groupement ester protecteur du radical hydroxy et X et les traits ondulés ont la signification indiquée précédemment,
que l'on soumet à l'action d'un agent de déshydrohalogénation pour obtenir le composé de formule (VII) : 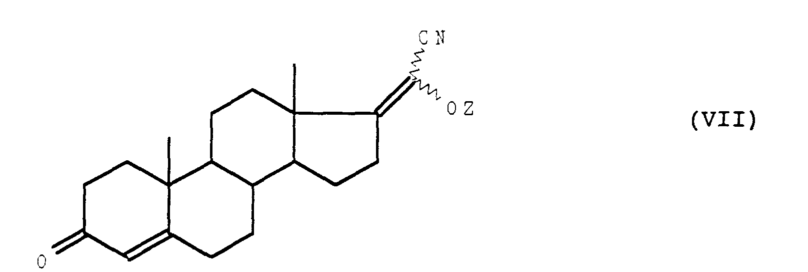 dans laquelle Z et les traits ondulés ont la signification indiquée précédémment, que l'on soumet à une hydrolyse alcaline puis à un traitement acide pour obtenir le composé de formule (I) attendu.

2. Procédé selon la revendication 1 **caractérisé en ce que** dans le composé de formule (II), les cycles A et B représentent un reste 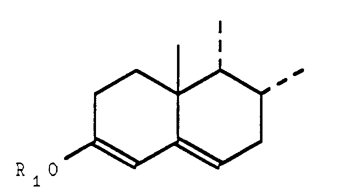 dans laquelle R₁ est défini comme à la revendication 1.

3. Procédé selon la revendication 2 **caractérisé en ce que** R₁ représente un radical alkyle renfermant de 1 à 6 atomes de carbone.

4. Procédé selon l'une quelconque des revendications 1 à 3 **caractérisé en ce que** l'on fait agir sur le composé de formule (II) le chloroacétonitrile, en présence d'un alcoolate de métal alcalin.

5. Procédé selon l'une quelconque des revendications 1 à 4 **caractérisé en ce que** l'hydracide utilisé pour l'ouverture de la fonction époxyde est l'acide chlorhydrique et que l'on opère en milieu anhydre, au sein d'un solvant organique non polaire.

6. Procédé selon la revendication 5, **caractérisé en ce que** l'on opère en présence d'une amine tertiaire.

7. Procédé selon l'une quelconque des revendications 1 à 6 **caractérisé en ce que** la protection de la fonction hydroxy en position 20 est effectuée sous forme d'ester d'un acide alcanoïque ou aromatique.

8. Procédé selon l'une quelconque des revendications 1 à 7 **caractérisé en ce que** la déshydrohalogénation est effectuée par action d'une amine tertiaire.

9. Procédé selon l'une quelconque des revendications 1 à 8, **caractérisé en ce que** l'hydrolyse alcaline est effectuée au moyen d'une base forte en présence d'eau ou d'un mélange eau-alcool.

10. Procédé selon l'une quelconque des revendications 1 à 9, **caractérisé en ce que** l'on opère sans isoler intermédiairement les composés de formule (IV) et suivantes.

11. Les composés de formule (F1) : 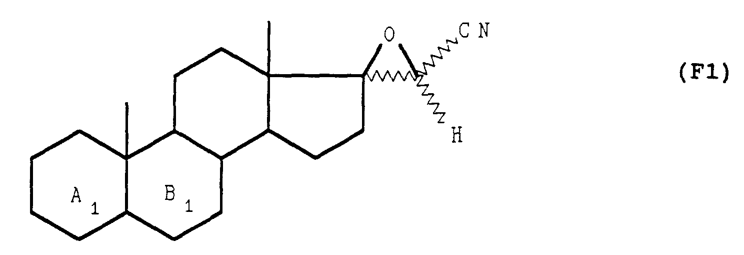 dans laquelle les cycles A1 et B1 ont la signification indiquée à la revendication 1 pour les cycles A et B, ou représentent le reste 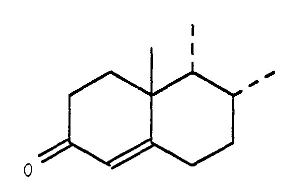 et les traits ondulés ont la signification indiquée à la revendication 1.

12. Les composés de formule (F2) : 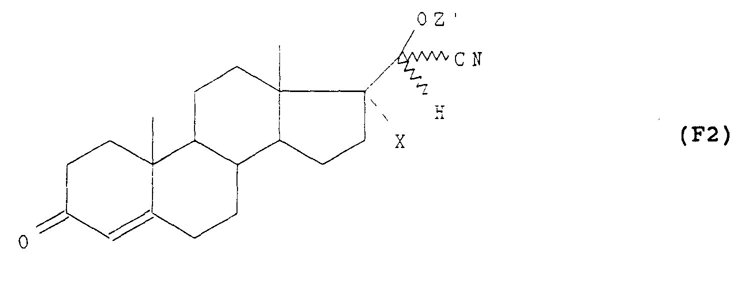 dans laquelle X et les traits ondulés ont la signification indiquée à la revendication 1 et Z' représente un atome d'hydrogène ou un groupement ester protecteur du radical hydroxy.

13. Les composés de formule (VII) : 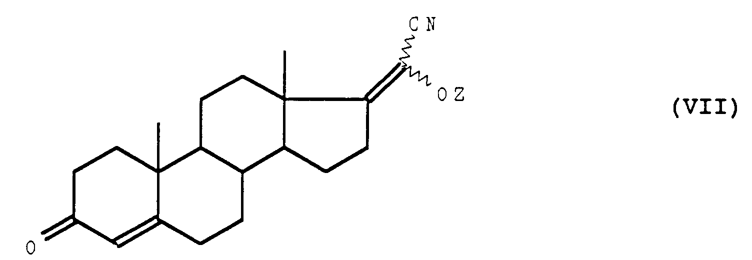 dans laquelle Z et les traits ondulés ont la signification indiquée à la revendication 1.

## Patentansprüche

1. Verfahren zur Herstellung von 0xoetiocholensäure der Formel (I): 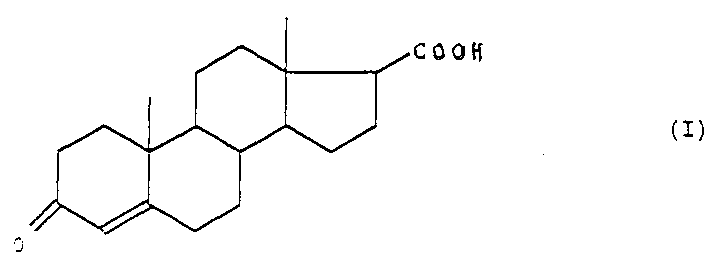 **dadurch gekennzeichnet, daß** man eine Verbindung der Formel (II): 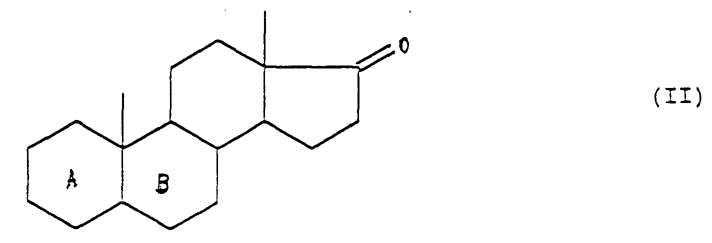 worin die Ringe A und B einen Rest: 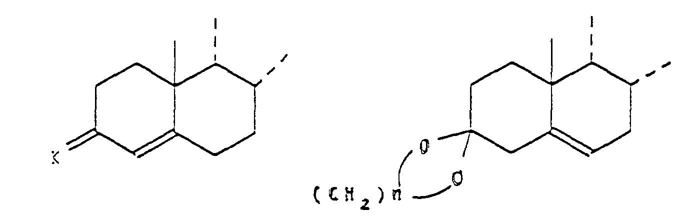 oder 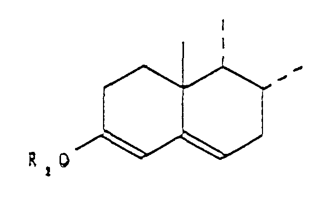 wiedergeben, worin K eine Schutzgruppe des Oxorestes der Formel: 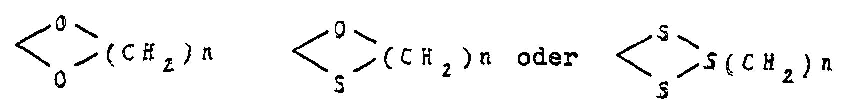 darstellt, worin n für 2 oder 3 steht und R₁ einen Ether- oder Esterrest bedeutet, der Einwirkung eines Halonitrils in Gegenwart einer Base unterzieht, um zu einer Verbindung der Formel (III): 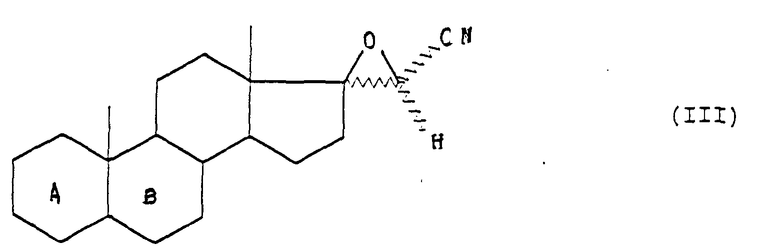 zu gelangen, worin die Ringe A und B die vorstehend angegebene Bedeutung besitzen und die gewellten Linien ein Isomerengemisch symbolisieren, woraus man die Ketonfunktion in 3-Stellung freisetzt, um zu einer Verbindung der Formel (IV): 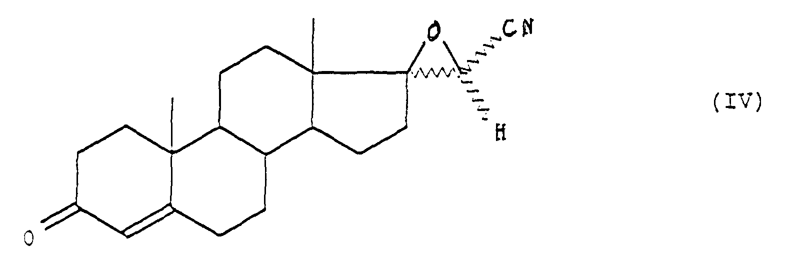 zu gelangen, worin die gewellten Linien die vorstehend angegebene Bedeutung haben,
welche Verbindung man mit einer Wasserstoffsäure der Formel HX, worin X für ein Halogenatom steht, in wäßrigem Milieu behandelt, um zu einer Verbindung der Formel (V): 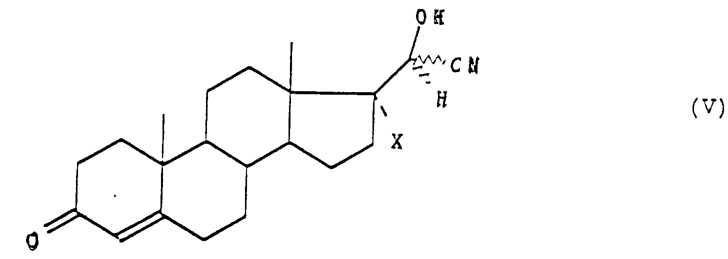 zu gelangen, worin X und die gewellten Linien die vorstehend angegebene Bedeutung besitzen,
deren Hydroxyfunktion man in Form des Esters schützt, um zu einer Verbindung der Formel (VI): 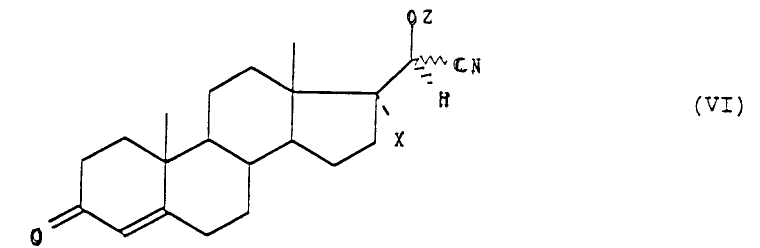 zu gelangen, worin Z den Rest einer Esterschutzgruppe des Hydroxyrestes wiedergibt und X und die gewellten Linien die vorstehend angegebene Bedeutung besitzen,
welche Verbindung man der Einwirkung eines Dehydrohalogenierungsmittels unterzieht, um zu der Verbindung der Formel (VII): 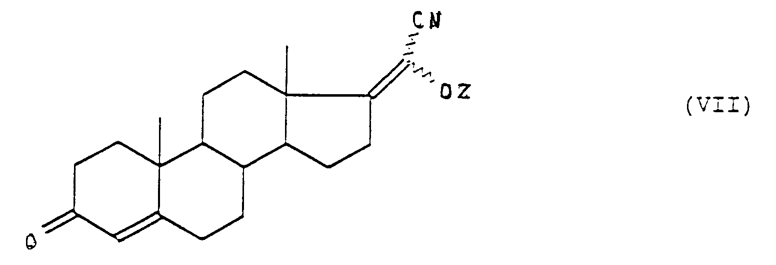 zu gelangen, worin Z und die gewellten Linien die vorstehend angegebene Bedeutung besitzen,
welche Verbindung man einer alkalischen Hydrolyse und danach einer sauren Behandlung unterzieht, um zu der erwarteten Verbindung der Formel (I) zu gelangen.

2. Verfahren gemäß Anspruch 1, **dadurch gekennzeichnet, daß** in der Verbindung der Formel (II) die Ringe A und B einen Rest 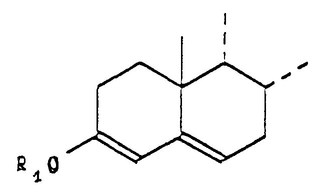 wiedergeben, worin R₁ wie in Anspruch 1 definiert ist.

3. Verfahren gemäß Anspruch 2, **dadurch gekennzeichnet, daß** R₁ einen Alkylrest mit 1 bis 6 Kohlenstoffatomen bedeutet

4. Verfahren gemäß einem der Ansprüche 1 bis 3, **dadurch gekennzeichnet, daß** man mit der Verbindung der Formel (II) Chloracetonitril in Anwesenheit eines Alkalimetallalkoholats umsetzt.

5. Verfahren gemäß einem der Ansprüche 1 bis 4, **dadurch gekennzeichnet, daß** die für die Öffnung der Epoxidfunktion verwendete Wasserstoffsäure Chlorwasserstoffsäure ist und daß man in wäßrigem Milieu in einem nicht-polaren organischen Lösungsmittel arbeitet.

6. Verfahren gemäß Anspruch 5, **dadurch gekennzeichnet, daß** man in Gegenwart eines tertiären Amins arbeitet.

7. Verfahren gemäß einem der Ansprüche 1 bis 6, **dadurch gekennzeichnet, daß** der Schutz der Hydroxyfunktion in 20-Stellung in Form des Esters einer Alkansäure oder aromatischen Säure erfolgt.

8. Verfahren gemäß einem der Ansprüche 1 bis 7, **dadurch gekennzeichnet, daß** die Dehydrohalogenierung durch Umsetzen mit einem tertiären Amin erfolgt.

9. Verfahren gemäß einem der Ansprüche 1 bis 8, **dadurch gekennzeichnet, daß** die alkalische Hydrolyse mit Hilfe einer starken Base in Gegenwart von Wasser oder eines Wasser-Alkohol-Gemisches erfolgt.

10. Verfahren gemäß einem der Ansprüche 1 bis 9, **dadurch gekennzeichnet, daß** man ohne intermediäre Isolierung der Verbindungen der Formel (IV) und der folgenden arbeitet.

11. Verbindungen der Formel (F1): 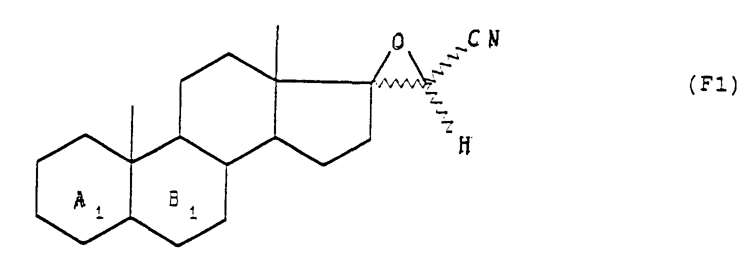 worin die Ringe A1 und B1 die in Anspruch 1 für die Ringe A und B angegebenen Bedeutungen besitzen oder den Rest 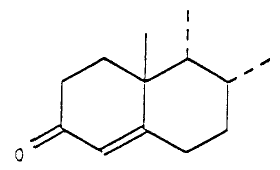 wiedergeben und die gewellten Linien die in Anspruch 1 angegebene Bedeutung haben.

12. Verbindungen der Formel (F2): 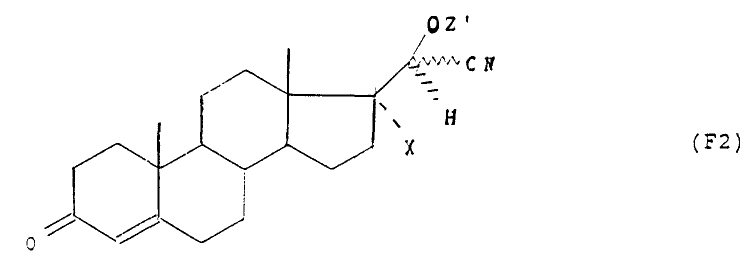 worin X und die gewellten Linien die in Anspruch 1 angegebene Bedeutung besitzen und Z' ein Wasserstoffatom oder eine Esterschutzgruppe des Hydroxyrestes bedeutet.

13. Verbindungen der Formel (VII): 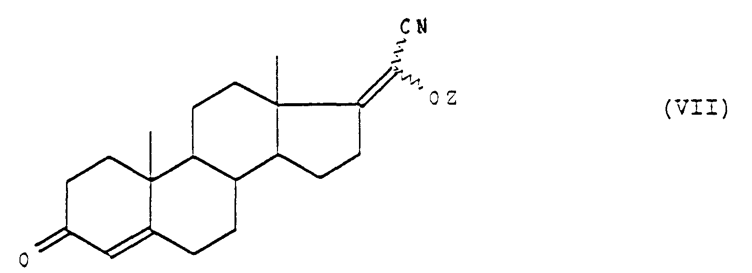 worin Z und die gewellten Linien die in Anspruch 1 angegebene Bedeutung besitzen.

## Claims

1. Process for the preparation of the oxoetiocholenic acid of formula (I): 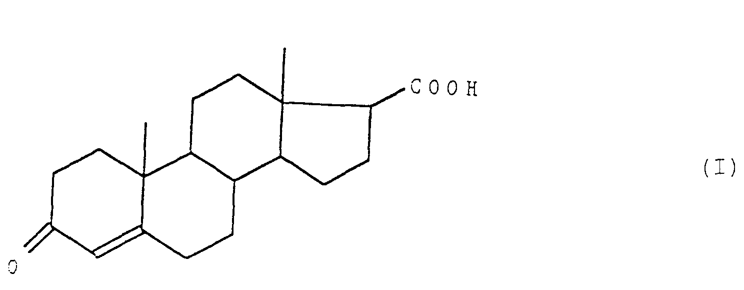 **characterized in that** a compound of formula (II) 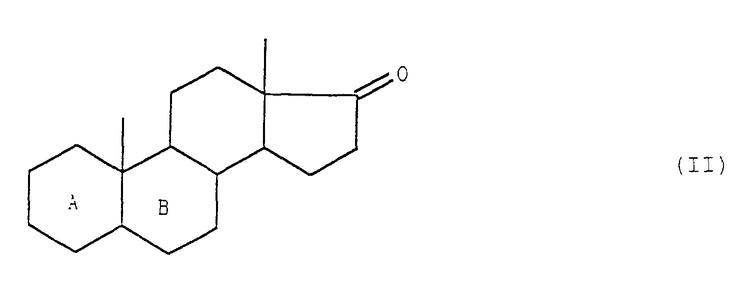 in which the rings A and B represent a remainder: 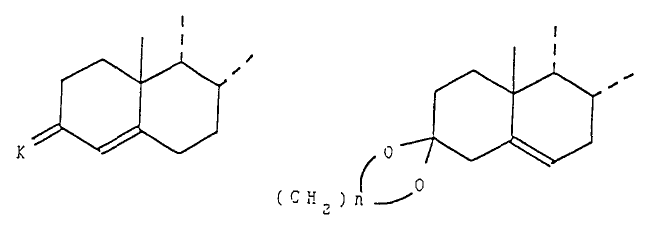 or 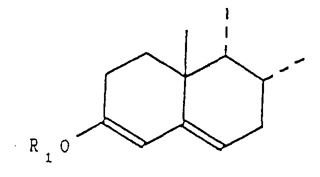 in which K represents a protective group of the oxo radical of formula: 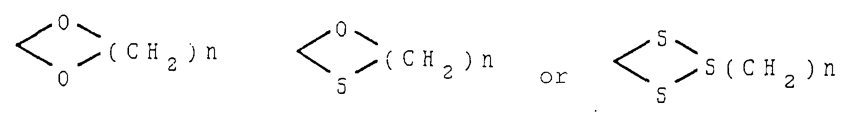 where n is equal to 2 or 3, and R₁ represents an ether or ester remainder, is subjected to the action of a halonitrile, in the presence of a base, in order to obtain a compound of formula (III): 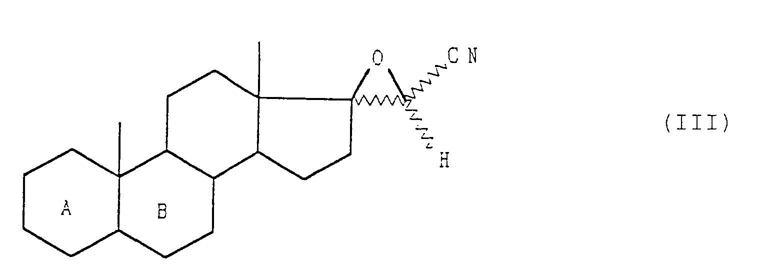 in which the rings A and B have the meaning indicated previously and the wavy lines symbolize a mixture of isomers, the free ketone function in position 3 of which is released in order to obtain a compound of formula (IV): 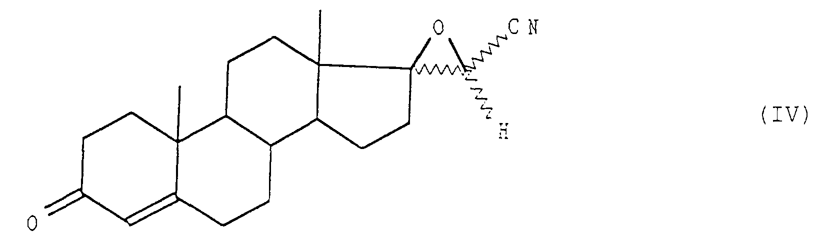 in which the wavy lines have the meaning indicated previously,
which is treated with a hydracid of formula HX in which X represents a halogen atom, in anhydrous medium, in order to obtain a compound of formula (V): 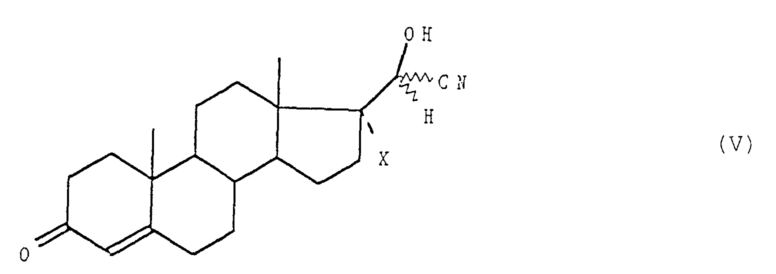 in which X and the wavy lines have the meaning indicated previously,
the hydroxy function of which is protected in the form of ester in order to obtain a compound of formula (VI): 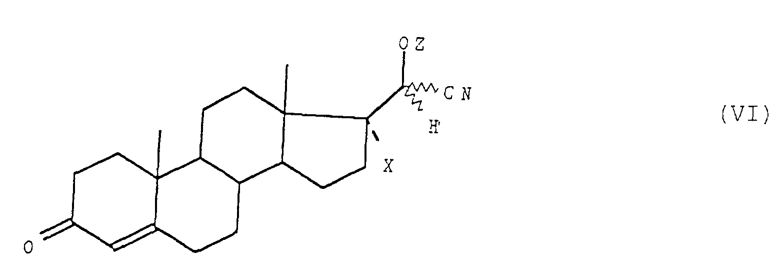 in which Z represents the remainder of an ester protective group of the hydroxy radical and X and the wavy lines have the meaning indicated previously,
which is subjected to the action of a dehydrohalogenation agent in order to obtain the compound of formula (VII) : 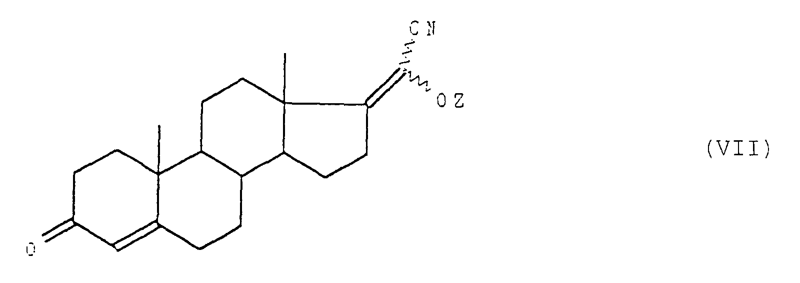 in which Z and the wavy lines have the meaning indicated previously,
which is subjected to an alkaline hydrolysis then to an acid treatment in order to obtain the expected compound of formula (I).

2. Process according to claim 1 **characterized in that** in the compound of formula (II), the rings A and B represent a 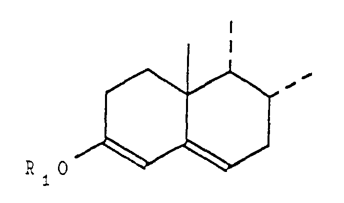 remainder in which R₁ is as defined in claim 1.

3. Process according to claim 2 **characterized in that** R₁ represents an alkyl radical containing 1 to 6 carbon atoms.

4. Process according to any one of claims 1 to 3 **characterized in that** chloroacetonitrile is reacted on the compound of formula (II), in the presence of an alkali metal alcoholate.

5. Process according to any one of claims 1 to 4 **characterized in that** the hydracid used for opening the epoxide function is hydrochloric acid and that the operation is carried out in anhydrous medium, in a non polar organic solvent.

6. Process according to claim 5, **characterized in that** the operation is carried out in the presence of a tertiary amine

7. Process according to any one of claims 1 to 6 **characterized in that** protection of the hydroxy function in position 20 is carried out in the form of an ester of an alkanoic or aromatic acid.

8. Process according to any one of claims 1 to 7 **characterized in that** the dehydrohalogenation is carried out by the action of a tertiary amine.

9. Process according to any one of claims 1 to 8, **characterized in that** the alkaline hydrolysis is carried out using a strong base in the presence of water or of a water-alcohol mixture.

10. Process according to any one of claims 1 to 9, **characterized in that** the operation is carried out without intermediate isolation of the compounds of formula (IV) and subsequent.

11. The compounds of formula (F1): 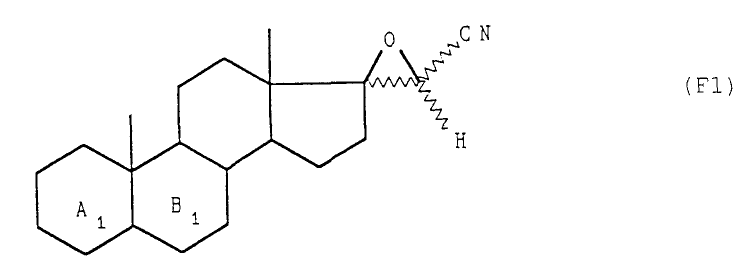 in which the rings A1 and B1 have the meaning indicated in claim 1 for the rings A and B, or represent the 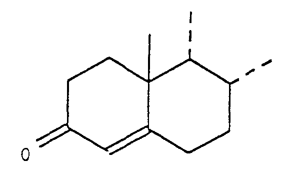 remainder and the wavy lines have the meaning indicated in claim 1.

12. The compounds of formula (F2): 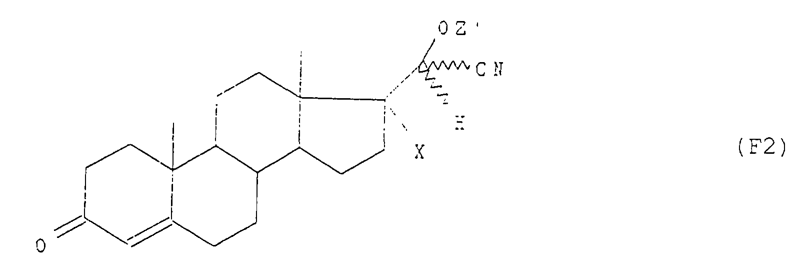 in which X and the wavy lines have the meaning indicated in claim 1 and Z' represents a hydrogen atom or an ester protective group of the hydroxy radical.

13. The compounds of formula (VII): 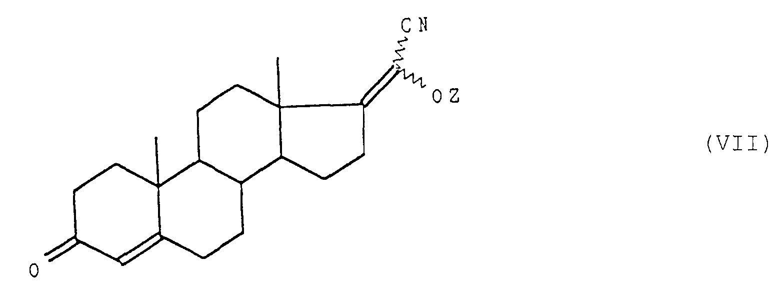 in which Z and the wavy lines have the meaning indicated in claim 1.
